Europäisches Patentamt

European Patent Office

Office européen des brevets

(11) Publication number: **0 316 995**

**A1**

## EUROPEAN PATENT APPLICATION

(21) Application number: **88202501.8**

(22) Date of filing: **09.11.88**

(51) Int. Cl.4: **A61N 1/05 , A61N 1/06 , A61B 17/39**

(30) Priority: **13.11.87 SE 8704458**

(43) Date of publication of application:
**24.05.89 Bulletin 89/21**

(84) Designated Contracting States:
**DE FR GB IT**

(71) Applicant: **Nordenström, Björn**
**Barrtorpsvägen 9**
**S-144 00 Rönninge(SE)**

(72) Inventor: **Nordenström, Björn**
**Barrtorpsvägen 9**
**S-144 00 Rönninge(SE)**

(74) Representative: **Bäckström, Leif C.**
**Protector Leif C. Bäckström AB P.O. Box 5132**
**S-162 05 Vällingby(SE)**

(54) **Electrode device intended to be introduced into the body of a living being.**

(57) An electrode device intended to be temporarily introduced into the body of a living being and to essentially locally treat or electrically measure biological body tissue therein in order to, after performed treatment or measurement, be removed. Said electrode device includes an electrode part (1), provided with an opening to its centre and insertable into the body tissue to be treated or measured, and a supply part (2), which electrically seen is isolated from the remaining body parts, for supply of electricity to said electrode part (1), which supply part (2) is made pliable and contains at least one channel.

Said electrode part (1) has an unlimited number of openings to its centre, which openings preferably are evenly distributed along the entire electrode part (1).

Fig. 3

EP 0 316 995 A1

## Electrode device intended to be introduced into the body of a living being

The invention relates to an electrode device intended to be temporarily introduced into the body of a living being and essentially locally treat or electrically measure biological body tissue therein in order to, after performed treatment or measurement, be removed, which electrode device includes an electrode part, provided with an opening to its centre and insertable into the body tissue to be treated or measured, and a supply part, which electrically seen is isolated from the remaining body parts, for supply of electricity to said electrode part, which supply part is made pliable and contains at least one channel.

Through, for example, Swedish patent 8002772-5, an electrode device is previously known for treatment of biological tissues inside a living being. Said known electrode device must - before introduction into the body - be adapted in size by adding one or more electrode rings in order to adapt the extension of the electrode to the size required.

Said known electrode device is, furthermore, provided with relatively small openings for supply of various agents via its central channel to the point of treatment and for removal of, for example, reaction products from the point of treatment. Said same openings and channel are also utilized for supply to as well as removal from the point of treatment, which circumstance makes a simultaneous supply and removal difficult or impossible to perform, for example in order to provide efficient cooling of the point of treatment.

The present invention aims at an electrode device which is more efficiently adaptable to the size required, preferably in situ at the point of treatment, and which electrode device provides improved transportation to and from the point of treatment and provides a much more enlarged effective electrode surface compared to previously known techniques.

The electrode device according to the preamble of the description is - in order to solve the above mentioned aim - designed in such a way that said electrode part has an unlimited number of openings to its centre, which openings preferably are evenly distributed along the entire electrode part.

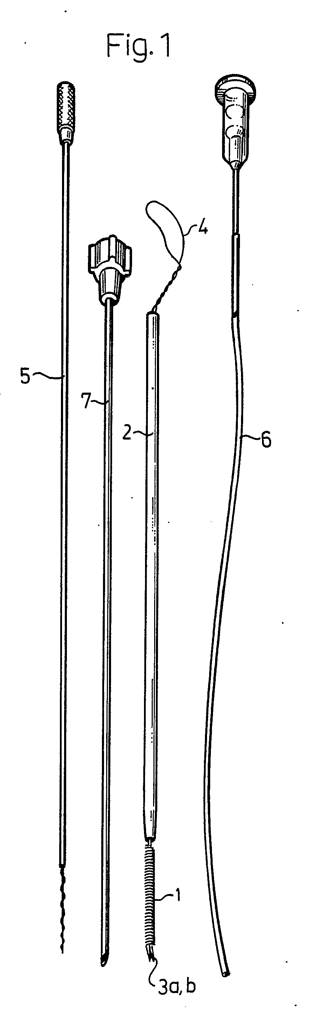

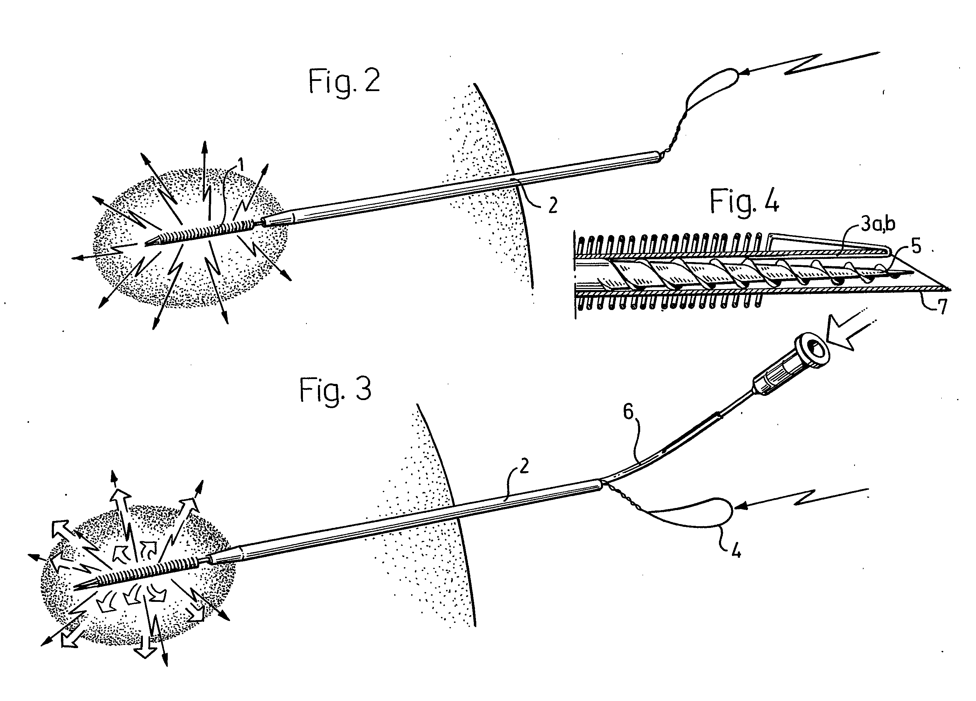

Other advantages with and features of the present invention appear more closely below and from the claims and from the enclosed drawings, in which drawings Fig. 1 shows a view over the various parts, which can be used when applicating and using the electrode device according to the invention, Fig. 2 shows the electrode device according to the invention during local treatment with electricity in situ in a living being, Fig. 3 shows the same device as shown in Fig. 2 but supplemented with an additional channel for transportation of liquids and/or gases to and from and for cooling of the point of treatment, respectively, and Fig. 4 shows a sectional view through the distal end of the electrode device.

The electrode part 1 in itself includes - according to the invention - one wire which in the one end 3 thereof preferably is bifilar wound and which wire preferably consists of platinum. The distal ends 3a, 3b of the wire are bent backwards, as is most evidently shown in Fig. 4, and the electrode wire runs through an electrically insulated tube 2, manufactured from teflon (trade name), for example, and the other end 4 of the wire runs through and projects out of the channel and is terminated by a loop. The wire 1 as well as the tube 2 are very flexible and can be bent and are, consequently, as such difficult or impossible to introduce into a living being.

In order to perform introduction of the electrode device into a living being, a cannula 7 consisting of, for example a stiff steel tube with an obliquely bevelled tip can be inserted into the tube 2 and running to the distal ends of said wire 3a, 3b, the said distal ends are arranged running into the channel of said cannula 7 a certain distance, which fact is particularly evident from Fig. 4. Furthermore, a screw means 5 having a threaded tip is introduced into the channel of said cannula 7 and is turned by means of its handle tip such that the threaded tip cooperates with said distal ends 3a, 3b and forces said distal ends against the inner wall of said cannula 7, whereby the electrode wire 1 is being clamped in the distal ends 3a, 3b in relation to the cannula 7 as well as to the screw means 5.

Hereby, the assembled unit has become a stiff unit and can, thus, be inserted into a living being to the point of treatment desired. When introduced, the distal ends 3a, 3b of the wire 1 are located in the position intended and the effective length of the electrode part 1 at the point of treatment is adapted to the size desired by pulling the loop-shaped end 4 of the electrode wire outside said living being having the distal ends 3a, 3b still clamped as specified above, the effective electrode part 1 is, thus, exactly adapted in length to the size desired.

When the above specificated manipulation has been performed, the screw means 5 can free the distal ends 3a, 3b by unscrewing said screw means 5. When the screw needle is free, the screw needle is used for pushing out the ends 3a, 3b from the tip of said cannula 7. The screw means 5 can, then, be removed as well as the cannula 7.

The electrode device is now ready to be used, for example, for treatment of the point of treatment with electricity as indicated in Fig. 2. From the point of view of the electrode, the comparatively very large effective electrode surface is to be noted and furthermore is to be noted that the electrode part 1 in itself as well as the electrically insulated tube 2 are very flexible and pliable and they, thereby, adjust themselves to movements in and around the point of treatment.

Through the tube 2, various agents can be supplied to and be removed from the point of treatment and communication is taking place via an infinite number of openings in the electrode part 1, which openings are located between the turns of the bifilar wound wire.

The electrode device according to the present invention can - if so desired or required - be supplemented with an extra, additional channel 6 in the shape of a flexible tube manufactured for, for example, teflon (trade name), which additional tube is inserted into the channel of said tube 2. Said additional channel 6 can preferably be introduced throughout the whole tube 2 and also a long distance into the electrode part 1, whereby a supply of, for example, cooling agents can be performed through said tube 6 and return transportation can be carried out between the outer wall of said tube 6 and the inner wall of said tube 2, by means of which a very effective mode of transportation - a circulating transportation - is obtained to and from the point of treatment without any essential obstacles.

When the desired use of the electrode device is completed, the extra channel 6 i firstly removed, whereafter the loop 4 is pulled so that the electrode part 1 is straightened out towards and is running through the tube 2 without damaging surrounding tissues, whereafter the tube 2 is removed by pulling it out.

## Claims

1. Electrode device intended to be temporarily introduced into the body of a living being and to essentially locally treat or electrically measure biological body tissue therein in order to, after performed treatment or measurement, be removed, which electrode device includes an electrode part (1), provided with an opening to its centre and insertable into the body tissue to be treated or measured, and a supply part (2), which electrically seen is isolated from the remaining body parts, for supply of electricity to said electrode part (1), which supply part (2) is made pliable and contains at least one channel, **characterized** in that said electrode part (1) has an unlimited number of openings to its centre, which openings preferably are evenly distributed along the entire electrode part (1).

2. Electrode device according to Claim 1, **characterized** in that the electrode part (1) is variable in size in relation to and inside the body tissue to be treated or measured.

3. Electrode device according to Claim 2, **characterized** in that the one, distal end (3) of the electrode part (1) is releaseably fixed at the electrode device and in that the second end (4) of the electrode part (1) is movable.

4. Electrode device according to Claim 3, **characterized** in that the electrode part (1) is changeable by adjusting the length thereof.

5. Electrode device according to anyone of previous Claims, **characterized** in that the electrode part is made as a spirally wound wire.

6. Electrode device according to anyone of previous Claims, **characterized** in that the electrode part (1) is made as a bifilar wound, screw-shaped cable, the one, distal end (3) of which cable comprises two wire ends (3a, 3b), which are clamped to the electrode device, and the other end of which cable is running through said channel of the isolated supply part (2) and out to the surroundings and is preferably arranged as a grip (4).

7. Electrode device according to anyone of the previous Claims, **characterized** in that said channel in size is so adapted that it allows for the introduction of an extra channel, which extra channel has such a length that said extra channel can be brought to emerge in the position desired in the centre of the electrode part.

8. Electrode device according to anyone of the previous Claims, **characterized** in that the electrode part is made from a thin wire or thread in order to be pliably straightened out during removal thereof.

Fig. 1

4

5

7

2

1

3a, b

6

Fig. 2

Fig. 4

1

2

3a,b

5

7

6

2

Fig. 3

4

EP 0 316 995 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| X | DE-A-3 516 830 (HUBMANN) <br> * Page 7, line 25 - page 12, line 29; figures 1-6 * | 1 | A 61 N 1/05 <br> A 61 N 1/06 <br> A 61 B 17/39 |
| A | | 2,4,7 | |
| X | DE-A-3 038 885 (FRESENIUS) <br> * Page 7, line 15 - page 9, line 4; figures 1,2 * | 1 | |
| A | | 2 | |
| D,A | WO-A-8 102 839 (URSUS KONSULT AB) <br> * Page 2, line 22 - page 7, line 26; figures 1-5 * & SE-A-8 002 772 | 1,2,4,7 | |
| A | WO-A-8 502 779 (BOARD OF TRUSTEES OF LELAND STANDORD JUNIOR UNIVERSITY) <br> * Page 4, line 29 - page 7, line 29; figures 1-4 * | 1,2,4,5 | |
| A | US-A-4 565 200 (COSMAN) | | |

**TECHNICAL FIELDS SEARCHED (Int. Cl.4)**

A 61 N
A 61 B
A 61 F

The present search report has been drawn up for all claims

| Place of search | Date of completion of the search | Examiner |
|---|---|---|
| THE HAGUE | 08-02-1989 | SCHMIERER U.J. |

EPO FORM 1503 03.82 (P0401)